# EUROPEAN PATENT APPLICATION

(11) **EP 2 275 111 A1**
(43) Date of publication of application: **19.01.2011**
(21) Application number: 09723216.9
(22) Date of filing: 17.03.2009
(51) Int. Cl.: A61K 36/07, A23L 1/30, A23L 1/305, A61K 31/715

(54) **ENTERAL NUTRIENT**

(30) Priority: 18.03.2008 JP 2008070295
(71) Applicant: Amino Up Chemical Co., Ltd., Hokkaido 004-0839 (JP)
(72) Inventor: FUJIKAWA, Keiko, Sapporo-shi Hokkaido 060-8638 (JP)
(74) Representative: Strehl Schübel-Hopf & Partner
(86) International application number: PCT/JP2009/055093
(87) International publication number: WO 2009/116507

(57) **Abstract**

The present invention relates to an enteral nutrient for inflammatory bowel disease patients, including an active hexose correlated compound obtained by culturing mycelia of basidiomycetes. The enteral nutrient of the present invention is an enteral nutrient which is expected to exhibit an advantageous effect of AHCC on cancer patients and an action of alleviating inflammation in inflammatory bowel diseases, and to exhibit an action of inhibiting inflammation in patients who are suffering fromulcerative colitis or Crohn' s disease and thus have difficulty with oral nutritional intake.

## Description

### Technical Field

The present invention relates to an enteral nutrient including an active hexose correlated compound obtained by culturing mycelia of basidiomycetes, which is beneficial for patients who are suffering from inflammatory bowel diseases or who are more likely to show symptoms of such diseases.

### Background Art

An active hexose correlated compound is a generic name for compositions including acetylated α-glucan and other polysaccharides obtained by culturing mycelia of basidiomycetes in large-scale tanks for a long period of time and extracting the culture. A representative example of the active hexose correlated compound is AHCC (registered trademark, the abbreviated name of the active hexose correlated compound) manufactured and sold by Amino Up Chemical Co., Ltd. (http://www.aminoup.co.jp/index.shtml).

The active hexose correlated compound (hereinafter, abbreviated as AHCC (registered trademark)) is one of substances each having an immunostimulatory function called "biological response modifiers mainly involved in immune responses" (biological response modifiers or BRMs) and has a potential to ameliorate lifestyle-related diseases such as cancer. Therefore, the active hexose correlated compound is one of functional polysaccharides on which the most advanced basic studies and clinical trials have been made.

An AHCC-containing health food is a food whose clinical effects have been pharmaceutically and medically confirmed by doctors as well as by a number of pharmaceutical and medical studies. In particular, the intake of an AHCC-containing food by patients who are being treated with an anticancer agent can reduce adverse effects such as myelosuppression (leucopenia and erythropenia), diarrhea, vomiting, anorexia, and alopecia, which are known as adverse effects of an anticancer agent. Accordingly, the AHCC-containing food has been utilized in a health food for improving the QOL of cancer patients.

However, there is a situation that AHCC is hard to be effectively ingested by patients who have difficulty with oral nutritional intake for the reasons of the original site of cancer and perioperative period.

A nutrient to be used in the case where oral nutritional intake is hard to be attained for various reasons is an enteral nutrient. The enteral nutrient is a nutrient used for directly supplying a nutritional substance into the stomach or intestine in older adults and infants who are generally likely to have reduced oral nutritional intake, patients with impaired oral intake, perioperative patients on dietary restrictions, and the like. The enteral nutrient must contain a sufficient amount of various nutritional components necessary for humans such as a carbohydrate, a protein, a fat, a mineral, a vitamin, and water in a well-balanced manner. Further, the enteral nutrient is prepared into an emulsion for easy feeding.

In these days, there are increasing needs for a novel enteral nutrient obtained by purposely adding, to an enteral nutrient, an active component capable of improving physical conditions of patients who take the nutrient as well as supplementing nutrition, which should also be referred to as a functional enteral nutrient. For example, JP 2006-50935 A (Patent Document 1) discloses an enteral nutrient including chitin hydrolysate and/or chitosan hydrolysate expected to have an ameliorating action on arthralgia or preventive and therapeutic effects on decubitus. Further, JP 2007-230998 A (Patent Document 2) discloses an oral anti-inflammatory agent having an effect of inhibiting the production of TNF-α in the intestinal tract, characterized by containing an acidic xylooligosaccharide having an uronic acid residue in the xylooligosaccharide molecule.

Patent Document 1: JP 2006-50935 A
Patent Document 2: JP 2007-230998 A

### Disclosure of the Invention

### Problem to be solved by the Invention

An object of the present invention is to provide a novel form of an AHCC-containing food capable of supplying AHCC to patients who have difficulty with oral nutritional intake.

### Means for solving the Problem

The inventors of the present invention have made studies on the development of an AHCC-containing enteral nutrient for cancer patients who have difficulty with oral nutritional intake. As a result, the inventors have found that the enteral administration of AHCC or its polysaccharide fraction provides an anti-inflammatory effect, which is expected to allow the alleviation of various symptoms of inflammatory bowel diseases. Thus, the inventors have completed the following respective inventions:

(1) An enteral nutrient for inflammatory bowel disease patients, including an active hexose correlated compound obtained by culturing mycelia of basidiomycetes.
(2) The enteral nutrient for inflammatory bowel disease patients according to (1) above, in which the basidiomycetes are one kind or two or more kinds of basidiomycetes selected from the group consisting of *Lentinulla edodes, Grifola frondosa, Ganoderma lucidum, Ganoderma applanatum* and *Schizophyllum commune.*
(3) The enteral nutrient for inflammatory bowel disease patients according to (1) above, in which the active hexose correlated compound is AHCC (registered trademark) or its polysaccharide fraction.
(4) The enteral nutrient for inflammatory bowel disease patients according to (1) or (2) above for promoting the production of interleukin-10 and/or interleukin-6.
(5) The enteral nutrient for inflammatory bowel disease patients according to (1) or (2) above, further including a protein and/or a digest thereof, a lipid and a carbohydrate, in which the total calories of the enteral nutrient are 70 to 200 kcal/100 ml.

### Effects of the Invention

The enteral nutrient of the present invention is an enteral nutrient which is expected to exhibit an advantageous effect of AHCC on cancer patients and an action of alleviating inflammation in inflammatory bowel diseases, and to exhibit an action of inhibiting inflammation in patients suffering from ulcerative colitis and Crohn's disease. In particular, the enteral nutrient is directly administered to an intestinal tissue to exhibit the action of inhibiting inflammationmore effectively.

### Best Mode for carrying out the Invention

The present invention provides an enteral nutrient for inflammatory bowel disease patients, including AHCC or its polysaccharide fraction. The enteral nutrient of the present invention is an enteral nutrient capable of not only providing a satisfactory action of AHCC or its polysaccharide fraction on cancer patients but also providing an effect of alleviating inflammation in inflammatory bowel disease patients. The action and effect are not attributed to nutrient components typified by a protein and the like contained in the enteral nutrient, but is attributed to AHCC or its polysaccharide fraction serving as a functional additive in the enteral nutrient.

AHCC is not a mere extract of basidiomycetes but a polysaccharide mixture produced by culturing mycelia of basidiomycetes in liquid tanks for a long period of time, for example, for 45 to 60 days, and subjecting the culture to an enzymatic reaction step, a sterilization step, a concentration step and the like. AHCC is estimated to include acetylated α-glucan as a main component, and also includes β-glucan and others.

Examples of the basidiomycetes which may be used for the production of AHCC include *Lentinulla edodes* (common name: shiitake mushroom), *Agaricus bisporus* (common name: button mushroom), *Grifola frondosa* (common name: Hen of the Woods), *Pholiota nameko* (common name: nameko mushroom), *Pleurotus ostreatus* (common name: oyster mushroom), *Flammulina velutipes* (common name: golden needle mushroom) , *Ganoderma lucidum* (common name: lingzhi), *Auricularia auricula* (common name: Judas' ear fungus), *Ganoderma applanatum* (common name: artist's fungus), *Coriolus lucidum* (turkey tail), *Grifola umbellate* (common name: umbrella polypore), *Schizophyllum commune* (common name: Split Gill) and *Volvariella volvaceae* (common name: straw mushroom).

Those basidiomycetes may be used alone or in combination of several kinds thereof. Of those, it is preferred to use *Lentinulla edodes* (*common name:shiitake mushroom*), *Grifola frondosa* (common name: Hen of the Woods), *Ganoderma lucidum* (common name: lingzhi), *Ganoderma applanatum* (common name: artist's fungus) and *Schizophyllum commune* (common name: Split Gill).

The basidiomycetes as exemplified above are preferably cultured using a plant tissue raw material. The plant tissue raw material is not particularly limited as long as the material is derived from a plant tissue, and for example, sawdust may also be used. However, herbaceous plant-derived materials such as rice bran, wheat bran, bagasse, corn rhizome, rice straw, wheat or barley straw, and soybean cake are preferred. Those may be used alone or in appropriate combination of a plurality of kinds thereof. Further, in order that the plant tissue raw material is easily assimilated by the basidiomycetes, there may be used an extract obtained by preliminarily treating the plant tissue rawmaterial with cellulase, amylase, protease, pectinase, chitinase or the like, and extracting the treated material with hot water. The plant tissue raw material may be added in the early culture period or the late culture period, depending on the culture state of the basidiomycetes.

Further, various carbon sources or nitrogen sources may be added to the plant tissue raw material. Examples of the carbon sources include glucose, sucrose, maltose, saccharose, white superior soft sugar, muscovado, molasses, blackstrap molasses and malt extract. Examples of the nitrogen sources include meat extract, peptone, gluten meal, soybean flour, dry yeast, yeast extract, ammonium sulfate, ammonium tartrate and urea. In addition, as necessary, inorganic salts such as a sodium salt, a magnesium salt, a manganese salt, an iron salt, a calcium salt and a phosphoric acid salt and vitamins such as inositol, vitamin B1 hydrochloride, L-asparagine and biotin may be added.

The culture of the basidiomycetes using the above-mentioned medium may be performed in accordance with general conditions for culturing mesophiles, for example, conditions at a pH of 2 to 6 and at a temperature of 10 to 45°C or preferably 15 to 30°C. The culture time may be generally about 4 to 20 days depending on the amount of the basidiomycetes and the form of the plant tissue raw material. The basidiomycetes can be cultured for a long period of time of 40 to 60 days to produce more excellent AHCC.

After the culture, an enzyme such as cellulase, amylase, protease, pectinase or chitinase is added to the resultant culture. An enzymatic reaction is then performed under an optimum temperature condition for 2 to 20 hours to decompose the mycelia of the basidiomycetes. After that, a heat treatment is performed to inactivate the enzymatic reaction, and a mycelium residue is removed from the treated product by centrifugation or the like to collect a supernatant fraction. Thus, an AHCC-containing fraction of interest can be collected.

Any AHCC may be employed as AHCC used in the present invention as long as it is obtained by selecting and culturing the mycelia of the basidiomycetes as exemplified above and subjecting the resultant culture to an enzymatic treatment. The quality of AHCC is likely to vary greatly depending on the production condition. Therefore, in order to keep high quality constant, the above-mentioned culture condition and collection and purification must be strictly maintained. At present, high-quality AHCCismanufactured and sold stably and continuously by Amino Up Chemical Co., Ltd. (http://www.aminoup.co.jp/index.shtml) in conformity with the standards according to the manufacture of pharmaceuticals. In the present invention, it is preferred to use a product marketed under AHCC (registered trademark) by Amino Up Chemical Co., Ltd.

Further, in the present invention, the polysaccharide fraction of AHCC may also be utilized together with AHCC or in place of AHCC. The "polysaccharide fraction of AHCC" in the present invention means a water-soluble fraction including polysaccharides derived from AHCC, wherein the fraction includes a water-soluble fraction in the case of subjecting AHCC to hydrophobic chromatography, a precipitate fraction obtained by adding ethanol to the water-soluble fraction, a non-adsorptive fraction in the case of subjecting the precipitate fraction to a cation chromatography treatment.

The blending amount of AHCC or its polysaccharide fraction in the enteral nutrient of the present invention is appropriately set in the range of 5 to 10 mass% with respect to the total mass of the nutrient. Further, any component generally used for an enteral nutrient may be used as the other components.

Examples of the protein include casein or casein salts such as casein sodium and casein calcium; animal proteins such as a milk protein, a chicken egg protein, a fish protein and a meat protein; vegetable proteins such as a soybean protein; and a decomposition product thereof and amino acids. Those may be used alone or in combination of two or more kinds thereof. The content of the protein is preferably 2.0 to 10.0 mass% or more preferably 3.5 to 6.0 mass% with respect to the mass of the enteral nutrient.

Examples of the lipid include vegetable oils such as soybean oil, corn oil, rapeseed oil, coconut oil, safflower oil, perilla oil, Japanese basil oil and palm oil; animal oils such as lard and beef tallow; fish oils; medium chain fatty acid triglycerides and other synthetic triglycerides; and processed oils thereof. Those may be used alone or in combination of two or more kinds thereof. In particular, with respect to fatty acids as a component of the lipid, it is preferred to blend a saturated fatty acid, a monounsaturated fatty acid and a polyunsaturated fatty acid in a well-balanced manner. Further, the content of the lipid is preferably 1.0 to 10.0 mass% or more preferably 2.0 to 8.0 mass% with respect to the mass of the enteral nutrient.

Examples of the carbohydrate include monosaccharides such as glucose and fructose; disaccharides such as maltose and lactose; oligosaccharides; and polysaccharides. Of those, it is preferred to use maltodextrin, dextrin, oligosaccharides or the like because of easy digestion and absorption and proper osmotic pressure. Those may be used alone or in combination of two or more kinds thereof. Further, the content of the carbohydrate is preferably 10.0 to 30 mass% or more preferably 15.0 to 25.0 mass% with respect to the mass of the enteral nutrient.

In order to sufficiently supply nutrition necessary for a human body, the enteral nutrient of the present invention preferably contains, in addition to the above-mentioned components, cellulose, polydextrose, enzymatically decomposed guar gum, hardly digestible polysaccharides and the like, dietary fiber, vitamins, and minerals, for example.

The total calories of the enteral nutrient of the present invention are 70 to 200 kcal/100 ml or more preferably 100 to 150 kcal/100 ml, and the amount of water to be added is appropriately adjusted so that the total calories fall within the above-mentioned range.

The enteral nutrient of the present invention preferably has a form of a solution, an emulsion or a soft jelly. In the emulsion, it is preferred to use an emulsifier such as a soybean phospholipid, an egg yolk phospholipid, a monoglyceride, a sucrose fatty acid ester, a sorbitan fatty acid ester, a propylene glycol fatty acid ester, a polyglycerin fatty acid ester, or a monoglyceride derivative such as a succinic acid monoglyceride or a citric acid monoglyceride. The content of the emulsifier is preferably 0.05 to 1.0 mass% or more preferably 0.2 to 0.7 mass% with respect to the total mass of the enteral nutrient.

The enteral nutrient of the present invention may be prepared in accordance with a conventional method. The solution may be prepared by dissolving blending components in water. Further, the emulsion may be prepared by dissolving blending components in water or preferably hot water, adding an oil and an emulsifier thereto, and emulsifying the mixture with a homogenizer or the like. The prepared solution and emulsion may be subjected to filter sterilization or hermetically filled into a pouch or a soft bag, and then subjected to heat sterilization to produce an enteral nutrient of the present invention.

The enteral nutrient of the present invention including AHCC or its polysaccharide fraction is expected to exert an effect of alleviating inflammation in inflammatory bowel diseases. As described in detail in Examples below, an effect of inducing the production of interleukin-10 (IL-10) as an anti-inflammatory cytokine was confirmed in a mammal to which AHCC or its polysaccharide fraction was administered enterally.

Further, it was confirmed that AHCC had an effect of inducing the production of interleukin-6 (IL-6) capable of inhibiting inflammation in a lipopolysaccharide (LPS) stimulation-free state. Moreover, it was confirmed that AHCC had an effect of inhibiting the production of interleukin-6, interleukin-2 (IL-2), TNF-α, and interleukin-12 (IL-12) as proinflammatory cytokines induced by LPS stimulation and the like. In addition, it was clarified that AHCC exhibited action of inhibiting the spreading inflammation on dendritic cells (DC) as sites of action.

Based on those physiological actions, the enteral nutrient of the present invention including AHCC or its polysaccharide fraction is expected to exert an effect of inhibiting inflammation in a variety of inflammatory bowel diseases such as Crohn's disease and ulcerative colitis. Further, the enteral nutrient is also effective with its anti-inflammatory ameliorating action on inflammatory bowel disease patients in a postoperative condition, an wound condition, or the like.

Hereinafter, the present invention is described in more detail by way of examples. However, the present invention is not limited to these examples.

### Examples

### Example 1:

80 mL of an AHCC (Amino Up Chemical Co., Ltd.) concentrate were charged into a column (5.0 cm i.d.x25 cm) filled with a polystyrene-type gel DIAION HP-20 (Mitsubishi Chemical Corporation) preliminarily conditioned with water. Elution was performed successively with 1 L each of water, methanol, and 50% acetone. The water fraction was concentrated under reduced pressure and then subjected to lyophilization to afford a yellowish white solid (18.1 g). To the fraction, 35 mL of water were added for dissolution. Then, 180 mL of ethanol were dropped thereto under vigorous stirring. After centrifugation (3000 rpm, 15 min), the supernatant was removed by decantation. 35 mL of water were added to the precipitate, followed by the same operations. To the resultant precipitate, a small amount of water was added for dissolution, and the solution was subjected to lyophilization to afford a light brown solid (5.87 g). To the fraction, 100 mL of water were added for dissolution, and the solution was charged into a column (4.4 cm i.d.×17 cm) filled with a cation-exchange-type gelDowex50WX-8(The Dow Chemical Company) . Elution was performed successively with 0.50 L each of water and 3 M aqueous ammonia. The water fraction was concentrated under reduced pressure and then subjected to lyophilization to afford a pale yellow solid (polysaccharide fraction of AHCC) (4.93 g).

### Example 2:

To 6-week-old C57BL/6 mice (female, n=30), AHCC (Amino Up Chemical Co., Ltd.) was administered with a gastric tube for consecutive 10 days (dosage of AHCC: 20 mg/1 ml/mouse/day). Two hours after intraperitoneal administration of LPS at 0.1 mg/2 mL/mouse on day 10, dendritic cells were separated from the spleen and the small intestine Peyer' s patches, and the production amounts of IL-10 were measured by a real-time PCR method using Applied Biosystems 7500 Real Time PCR System. It should be noted that a mouse group in which an enteral nutrient having the same composition as that in Example 2 except for being free of AHCC was administered in the same manner as described above was prepared as a control.

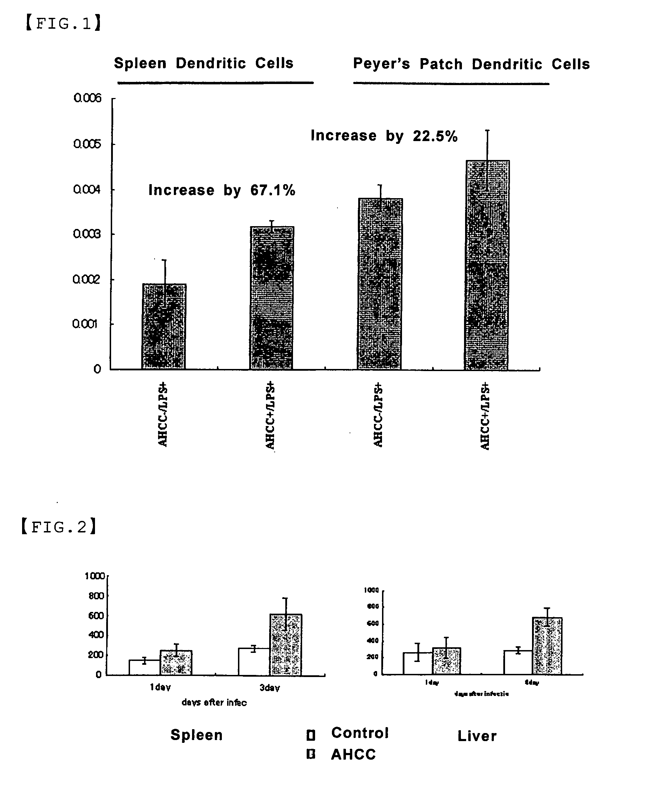

The results showed that the production amount of IL-10 in the dendritic cells separated from the spleen increased by 67.1% as compared to that in the control. The results also showed that the production amount of IL-10 in the dendritic cells separated from the small intestine Peyer' s patches increased by 22.5% as compared to that in the control (FIG. 1).

### Example 3:

To 8-week-old C57BL/6 mice (female, n=10), AHCC (Amino Up Chemical Co., Ltd.) was administered with a gastric tube for 15 consecutive days (dosage of AHCC: 20 mg/1 ml/mouse/day). On day 10, *Rhodococcus aurantiacus* was intravenously administered at 1×10⁸ CFU/0.2 mL/mouse. *Rhodococcus aurantiacus* is known as a microorganism which preferentially accumulates in spleen cells and liver cells. Thus, in order to study infectious conditions in the two organs, spleen and liver were collected on day 1 and day 3 after administration, and the expression amounts of IL-6 and IL-10 were measured using DuoSet ELISA Development System (R&D).

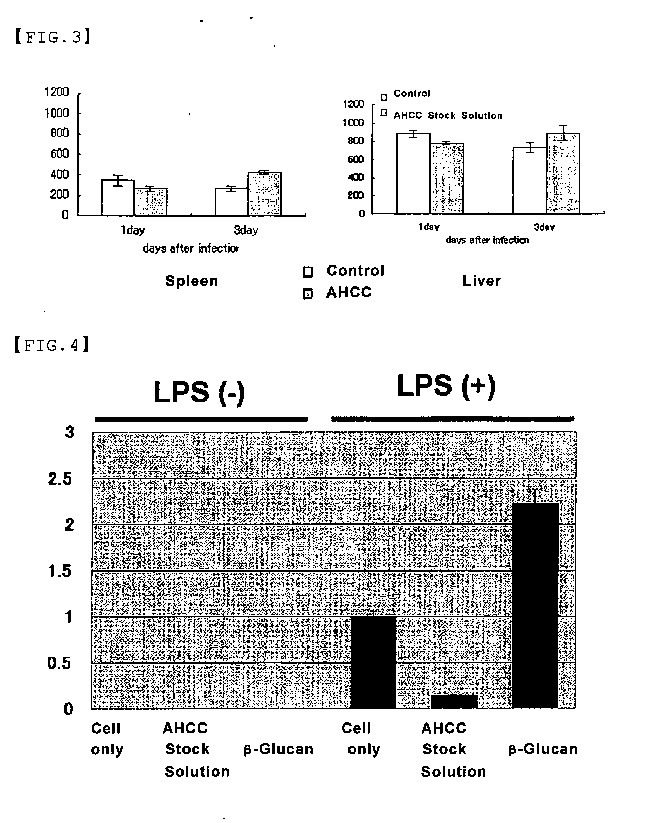

The results confirmed that both the production amounts of IL-6 (FIGS. 2) and the production amounts of IL-10 (FIGS. 3) increased in the spleen cells and the liver cells. In an infectious disease with *Rhodococcus aurantiacus,* it is known that both IL-10 and IL-6 are induced as anti-inflammatory cytokines. Accordingly, the above-mentioned experimental results suggest that AHCC promotes the production of IL-10 and IL-6 serving as anti-inflammatory cytokines, that is, AHCC has an anti-inflammatory action.

### Example 4:

To 8-week-old C57BL/6 mice (female, n=10), each of AHCC (Amino Up Chemical Co., Ltd.) or its polysaccharide fraction and β-glucan as a control was administered with a gastric tube for 15 consecutive days (dosage of AHCC or its polysaccharide fraction: 20 mg/1 ml/mouse/day). On day 3 after intraperitoneal administration of LPS at 0.1 mg/2 mL/mouse on day 10, spleen and liver were collected.

With the use of 1000 ng of the total RNA extracted from dendritic cells separated from the spleen and the liver as a template, RT-PCR was performed using AMV Reverse Transcriptase XL (Takara) and Random 9mer. In addition, with the use of cDNA obtained by RT-PCR as a template, the expression amounts of IL-2, IL-6, IL-10, and TNF-α were measured by real-time PCR (9500 Real Time Pcr System, Applied Biosystems) using the following primer sets specific for the respective cytokines.

- IL-2:: Forward 5'-GGAGCAGCTGTTGATGGACCTAC-3' (SEQ ID NO: 1)
Reverse 5'-AATCCAGAACATGCCGCAGAG-3' (SEQ ID NO: 2)
- IL-6:: Forward 5'-CAAGAAAGACAAAGCCAGAGTC-3' (SEQ ID NO: 3)
Reverse 5'-GGTTTGCCGAGTAGATCTCAA-3' (SEQ ID NO: 4)
- IL-10:: Forward 5'-AGCCTTATCGGAAATGATCCAG-3' (SEQ ID NO: 5)
Reverse 5'-TGCTCCACTGCCTTGCTCTTA-3' (SEQ ID NO: 6)
- TNF-α:: Forward 5'-AGAAGAGGCACTCCCCCAAAAG-3' (SEQ ID NO: 7)
Reverse 5'-GGCTACAGGCTTGTCACTCGAA-3' (SEQ ID NO: 8)

The expression amounts were quantified by using GAP-DH as a control, plotting the number of copies on the ordinate and the number of cycles on the abscissa, and determining the number of copies converted from the number of cycles. Further, the difference in total RNA amount was corrected by dividing the number of copies of the respective targets by the number of copies of GAP-DH.

The results confirmed that AHCC strongly inhibited the expression of IL-2 (FIG. 4), IL-6 (FIG. 5), and TNF-α (FIG. 6) induced by LPS stimulation. The results also confirmed that AHCC or its polysaccharide fraction had an action of inducing the production of IL-10 (FIG. 7).

### Example 5:

Spleen cells and liver cells were collected 5 hours after administration of *Rhodococcus aurantiacus,* and the expression amounts of IL-12 were measured by real-time PCR (9500 Real Time Pcr System, Applied Biosystems) using the following primer set.

- IL-12:: Forward 5'-CCAGAGACATGGAGTCATAGGC-3' (SEQ ID NO: 9)
Reverse 5'-CAAGTCCATGTTTCTTTGCACC-3' (SEQ ID NO: 10)

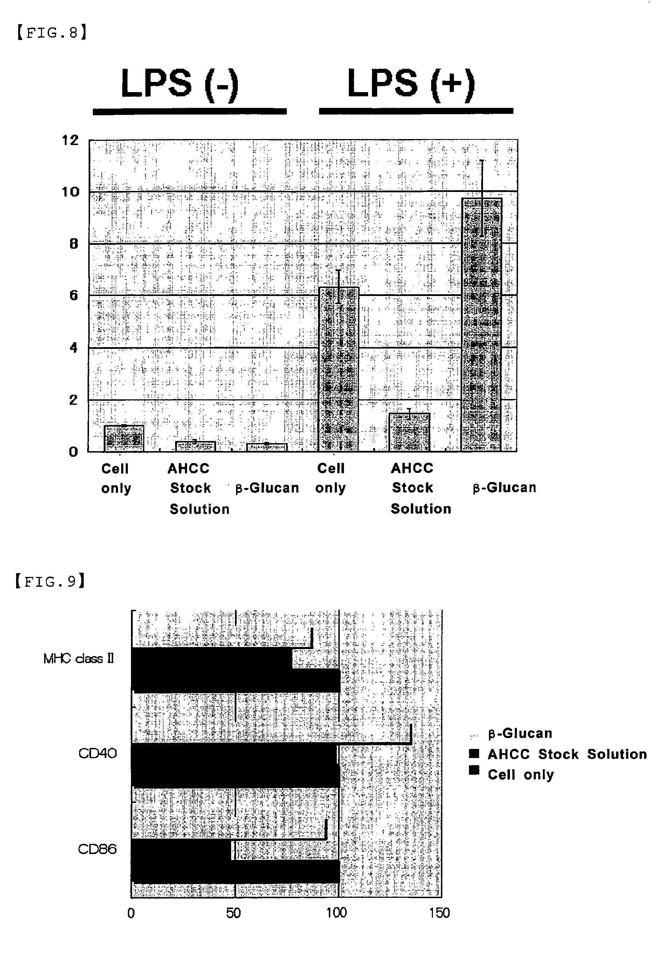

The results confirmed that AHCC strongly inhibited the expression of IL-12 induced by LPS stimulation (FIG. 8).

### Example 6:

Dendritic cells separated from the bone marrow and spleen of normal mice were placed in plastic dishes to measure the external appearances of dendritic cells in the case of adding only LPS so as to achieve a final concentration of 5 µg/ml and in the case of adding LPS and AHCC (final concentration: 5 mg/ml) or β-glucan (final concentration: 1 mg/ml). The table shows the results.

**[Table 1]**

| | Normal condition | | | LPS stimulation | | |
|---|---|---|---|---|---|---|
| | (-) | AHCC | β-glucan | (-) | AHCC | β-glucan |
| Cell morphology | Spherica 1shape | Spherica 1shape | Spherica 1shape | Deformation | Spherica 1shape | Deformation |
| Polarity | + | ± | - | +++ | - | ++ |
| Degree of accumulation | - | ++ | ± | ± | ++ | + |

Further, the degree of maturation of those dendritic cells was also measured by Facs analysis using the expression amounts of CD86, CD40, and an MHC class II molecule as indicators.
The results confirmed that the addition of AHCC dominantly inhibited the expression amounts of the above-mentioned three kinds of proteins induced by LPS addition, i.e., dendritic cells did not maturate even when being stimulated with LPS (FIG. 9). This means that AHCC can inhibit inflammation induced by LPS addition.

### Reference Example 1:

70 g of dextrin, 8 g of casein sodium, 12 g of AHCC (Amino Up Chemical Co. , Ltd.), and a small amount each of a vitamin mixture and inorganic salts were dissolved in hot water at 50°C. After that, 9 g of corn oil and 0.025 g of a sugar ester were added for emulsification to prepare an enteral nutrient having a total volume of 100 ml.

### Reference Example 2:

18 g of dextrin, 4 g of an egg white hydrolysate, an inorganic salt mixture, a vitamin mixture, and 4 g of AHCC (Amino Up Chemical Co., Ltd.) were dissolved in water so that the total volume was 100 ml. The resultant solution was then subjected to filter sterilization to afford a liquid enteral nutrient. The content of AHCC in the enteral nutrient was 20 mg/mL.

### Brief Description of the Drawings

FIG. 1 is a graph illustrating the enhancement of an ability to produce IL-10 in dendritic cells of mice that have received AHCC enterally.
FIGS. 2 are graphs each illustrating the enhancement of an ability to produce IL-6 in mice that have received AHCC enterally.
FIGS. 3 are graphs each illustrating the enhancement of a production ability of IL-10 in mice that have received AHCC enterally.
FIG. 4 is a graph illustrating that AHCC inhibits the production of IL-2 in mice stimulated with LPS.

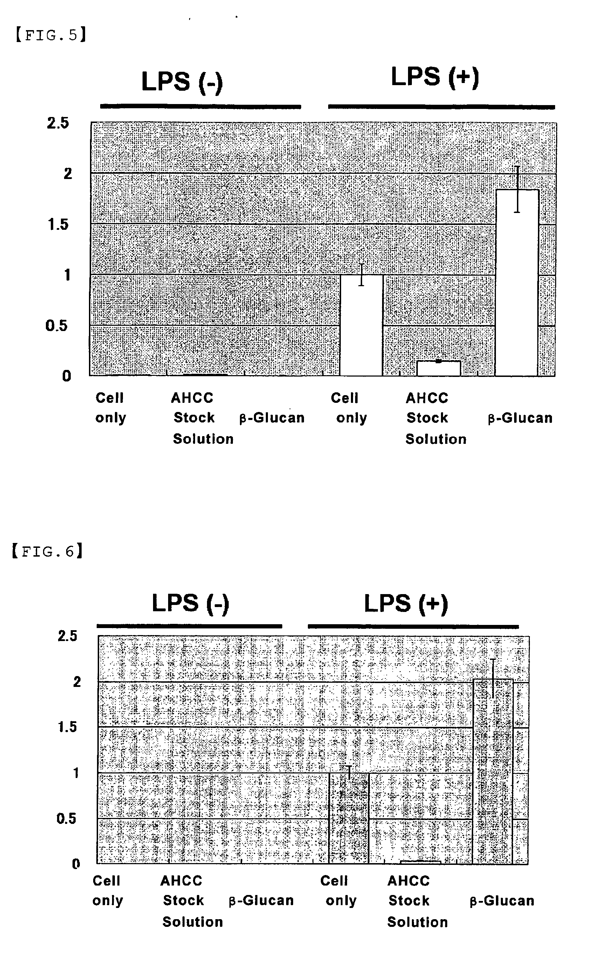

FIG. 5 is a graph illustrating that AHCC inhibits the production of IL-6 in mice stimulated with LPS.
FIG. 6 is a graph illustrating that AHCC inhibits the production of TNF-α in mice stimulated with LPS.

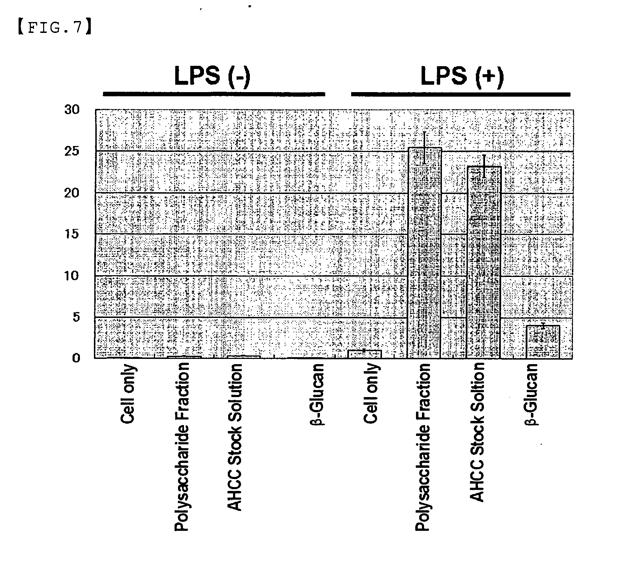

FIG. 7 is a graph illustrating the enhancement of an ability to produce IL-10 in mice stimulated with LPS.
FIG. 8 is a graph illustrating that AHCC inhibits the production of IL-12 in mice stimulated with LPS.
FIG. 9 is a graph illustrating that AHCC inhibits the expression induction of CD86, CD40, and an MHC class II molecule in dendritic cells stimulated with LPS.

## Claims

1. An enteral nutrient for inflammatory bowel disease patients, including an active hexose correlated compound obtained by culturing mycelia of basidiomycetes.

2. The enteral nutrient for inflammatory bowel disease patients according to claim 1, in which the basidiomycetes are one kind or two or more kinds of basidiomycetes selected from the group consisting of *Lentinulla edodes, Grifola frondosa, Ganoderma lucidum, Ganoderma applanatum* and *Schizophyllum commune.*

3. The enteral nutrient for inflammatory bowel disease patients according to claim 1, in which the active hexose correlated compound is AHCC (registered trademark) or its polysaccharide fraction.

4. The enteral nutrient for inflammatory bowel disease patients according to claims 1 or 2 for promoting the production of interleukin-10 and/or interleukin-6.

5. The enteral nutrient for inflammatory bowel disease patients according to claims 1 or 2, further including a protein and/or a digest thereof, a lipid and a carbohydrate, in which the total calories of the enteral nutrient are 70 to 200 kcal/100 ml.


### Sequence Listing

SEQUENCE LISTING

<110>  Amino Up Chemical Co., Ltd.

<120>  Enteral nutrition

<130>  AUF-7100PCT

<150>  JP2008-070295
<151>  2008-03-18

<160>  10

<170>  PatentIn version 3.3

<210>  1
<211>  23
<212>  DNA
<213>  Artificial sequence

<220>
<223> primer DNA

<400>  1
ggagcagctg ttgatggacc tac                                              23


<210>  2
<211>  21
<212>  DNA
<213>  Artificial sequence

<220>
<223> primer DNA

<400>  2
aatccagaac atgccgcaga g                                                21


<210>  3
<211>  22
<212>  DNA
<213>  Artificial sequence

<220>
<223> primer DNA

<400>  3
caagaaagac aaagccagag tc                                               22


<210>  4
<211>  21
<212>  DNA
<213>  Artificial sequence

<220>
<223> primer DNA

<400>  4
ggtttgccga gtagatctca a                                                21

<210>  5
<211>  22
<212>  DNA
<213>  Artificial sequence

<220>
<223> primer DNA

<400>  5
agccttatcg gaaatgatcc ag                                                22


<210>  6
<211>  21
<212>  DNA
<213>  Artificial sequence

<220>
<223> primer DNA

<400>  6
tgctccactg ccttgctctt a                                                 21


<210>  7
<211>  22
<212>  DNA
<213>  Artificial sequence

<220>
<223> primer DNA

<400>  7
agaagaggca ctcccccaaa ag                                                22


<210>  8
<211>  22
<212>  DNA
<213>  Artificial sequence

<220>
<223> primer DNA

<400>  8
ggctacaggc ttgtcactcg aa                                                22


<210>  9
<211>  22
<212>  DNA
<213>  Artificial sequence

<220>
<223> primer DNA

<400>  9
ccagagacat ggagtcatag gc                                                22


<210>  10
<211>  22
<212>  DNA

<213>  Artificial sequence

<220>
<223> primer DNA

<400>  10
caagtccatg tttctttgca cc                                                22